# FASCICULE DE BREVET EUROPEEN

(11) **EP 0 730 658 B1**
(45) Date de publication et mention de la délivrance du brevet: **20.03.2002**
(21) Numéro de dépôt: 95931271.1
(22) Date de dépôt: 20.09.1995
(51) Int. Cl.: C12P 7/48, A23L 1/0522, C13K 1/06, C13K 1/08

(54) **NOUVEAU PROCEDE DE FABRICATION DE L'ACIDE CITRIQUE**
VERFAHREN ZUR HERSTELLUNG VON ZITRONENSÄURE
NOVEL METHOD FOR PRODUCING CITRIC ACID

(30) Priorité: 23.09.1994 FR 9411388
(43) Date de publication de la demande: 11.09.1996
(73) Titulaire: Roquette Frères, 62136 Lestrem (FR)
(72) Inventeur: SANIEZ, Marie-Hélène, F-59350 Saint-André (FR)
(74) Mandataire: Boulinguiez, Didier
(86) Numéro de dépôt international: FR9501209
(87) Numéro de publication internationale: WO9609401

(56) Documents cités:
- EP-A- 0 136 087
- US-A- 2 492 673
- US-A- 2 970 084
- US-A- 4 165 240
- PREHRAMBENO - TEHNOLOSKA I BIOTEHNOLOSKA REVIJA, 1994, 32_(1) 17-20, COLNAR, C. ET AL 'Citric acid production on a waste starch fraction hydrolysate.'

## Description

L'invention a pour objet un procédé de fabrication de l'acide citrique par voie fermentaire.

Elle vise également et ce, à titre de produits industriels nouveaux, certaines matières premières qui peuvent être mises en oeuvre dans le cadre de cette fabrication.

Elle vise enfin l'application à ce procédé de certains produits amylacés.

L'acide citrique est utilisé principalement dans l'industrie alimentaire où son goût acide plaisant et sa grande solubilité le font apprécier surtout dans les boissons, les confitures et les friandises, mais il est également utilisé dans les industries pharmaceutiques, cosmétiques et dans les industries des plastiques et de la détergence.

L'acide citrique est désormais obtenu quasi exclusivement par des procédés fermentaires faisant appel à la fermentation en cuves profondes de divers substrats à base d'hydrates de carbone.

Les microorganismes capables de produire et d'accumuler l'acide citrique peuvent appartenir aux espèces Aspergillus, Citromyces, Penicillium, Monilia, Candida et Pichia. Les Aspergillus noirs et surtout Aspergillus niger sont particulièrement employés dans ce type de production. Les souches les plus efficaces sont celles qui possèdent des activités isocitrate déshydrogénase et aconitate hydratase faibles et une forte activité citrate synthase. Ces souches ont toutes en commun la particularité d'être très sensibles aux traces de métaux lourds.

Les hydrates de carbone auxquels ces procédés fermentaires font appel peuvent être plus ou moins raffinés. Cependant, leur état de pureté entraîne des contraintes différentes.

Ainsi le saccharose ou le dextrose cristallisés de qualité alimentaire ne contiennent pas de métaux lourds. Ils fournissent des rendements d'acide citrique supérieurs à 70 % mais sont onéreux.

Cependant, la récupération de l'acide citrique se trouve grandement facilitée par la pureté de tels substrats.

Les mélasses de canne ou de betterave sont des substrats peu, onéreux. Toutefois, la grande quantité d'impuretés qu'elles renferment posent d'énormes problèmes de traitement des métaux lourds et de purification de l'acide citrique.

Les hydrolysats d'amidon sont un peu plus onéreux que les mélasses. Qu'ils soient de maïs, de blé, de pomme de terre, de riz, ils contiennent moins d'impuretés que les mélasses et la purification de l'acide citrique s'en trouve facilitée. Cependant, leur emploi se trouve limité parce que ces substrats peu purifiés contiennent également des substances de nature inconnue qui augmentent les effets néfastes des métaux lourds sur la fermentation citrique.

On pense que ces substances qui potentialisent l'effet des métaux lourds que sont essentiellement le fer, le manganèse et le zinc, peuvent être des résidus d'acides aminés, des peptides, des enzymes ou des métabolites intermédiaires. A l'inverse, certaines substances stimulatrices de la fermentation citrique ont parfois été mises en évidence bien que leur nature chimique n'ait pas été éclaircie.

Il existe des méthodes qui permettent de lutter contre l'effet néfaste des métaux lourds présents dans les substrats peu purifiés, sur la fermentation citrique.

On peut par exemple les complexer à l'aide d'hexacyanoferrate ou contrebalancer leurs effets par l'addition de cuivre.

Le brevet US 2 970 084 décrit l'utilisation du cuivre en tant qu'antagoniste du fer dans la confection de milieux de culture impurs destinés à la fermentation citrique. Ces milieux impurs peuvent être des hydrolysats d'amidon en toute généralité.

Le brevet US 2 492 673 décrit la production d'acide citrique par fermentation de « mélasses inverties » par Aspergillus niger après passage desdites « mélasses inverties » sur colonnes échangeuses d'ions permettant de réduire le contenu en espèces Fe et d'ajouter un équivalent Zn à chaque Fe résiduel.

MOYER (J. App. Microbio 1, 7-13), à l'aide d'une souche d'Aspergillus niger douée de propriétés amylolytiques, démontra que l'on pouvait aussi fermenter avec succès un amidon natif de blé, non hydrolysé, en acide citrique et ce, en neutralisant l'effet des métaux lourds par l'addition d'un alcool.

SWARTHOUT (Brevet américain 3 285 831) employa un hydrolysat enzymatique d'une farine brute de maïs pour obtenir l'acide citrique par l'action d'Aspergillus niger. Il se débarrassa des métaux lourds en les piégeant sur une résine cationique et suggéra aussi de traiter de la sorte un hydrolysat enzymatique d'une farine de blé. De tels hydrolysats obtenus à partir de farines complètes de céréales, contiennent cependant beaucoup d'impuretés, et notamment beaucoup de protéines qui interfèrent avec la récupération finale de l'acide citrique.

Le brevet US 4 165 240 décrit une solution de dextrose débarrassée de ses métaux lourds par passage sur une résine chélatante et isomérisation d'au moins une part de ce dextrose en fructose.

La demande de brevet EP A-0 136 087 décrit un procédé de préparation de dextrose à partir d'un lait d'amidon sur colonne de chromotographie.

Cependant, on n'a pas encore identifié, ni tiré parti d'un substrat peu onéreux qui soit suffisamment pur pour ne pas occasionner de problèmes de récupération de l'acide citrique ou d'élimination des métaux lourds et qui en plus, contienne des substances stimulatrices de la fermentation citrique.

Le mérite de la Société Demanderesse est d'avoir reconnu un tel substrat dans un hydrolysat d'un sous-produit de l'amidonnerie de blé, couramment dénommé amidon B. De façon surprenante, un tel substrat permet d'obtenir des productivités et rendements bien plus élevés que ne le permet un hydrolysat obtenu à partir d'un amidon plus pur tel que l'amidon de blé A ou un amidon de mais par exemple.

En outre, le fait d'avoir recours à un hydrolysat d'amidon, plutôt qu'à un amidon natif, comme le faisait MOYER, autorise des productivités supérieures puisque la vitesse de la fermentation n'est pas limitée par le potentiel amylolytique des souches d'Aspergillus employées.

L'amidon B ou amidon second est constitué essentiellement par une proportion prépondérante de petits granules d'amidon ou de granules endommagés et contient des impuretés telles que des pentosanes, des protéines et des lipides.

Ces impuretés de l'amidon B de blé, dont certaines échappent aux traitements classiques de purification et de déminéralisation, se retrouvent dans les hydrolysats de ces amidons et rendent l'amidon B impropre par exemple, à la fabrication du dextrose de qualité alimentaire. De tels amidons B trouvent ainsi difficilement des débouchés industriels.

Cependant, et c'est ce qui est à la base de son invention, la Société Demanderesse a découvert que ces impuretés possèdent un effet bénéfique sur la fermentation citrique, offrant ainsi un débouché industriel nouveau à ce sous-produit.

L'amidon B est obtenu dans la proportion d'environ 6 à 17 Kgs d'amidon par quintal de farine de blé mise en oeuvre en amidonnerie, tandis que l'amidon A, plus pur, essentiellement composé de gros granules d'amidon est obtenu dans une proportion d'environ 55 à 65 Kgs d'amidon par quintal de farine de blé mise en oeuvre. Seule cette qualité d'amidon de blé a trouvé jusqu'à maintenant des applications nobles.

On trouvera un exposé des procédés couramment employés par l'amidonnerie de blé dans l'ouvrage : "STARCH : Chemistry and Technology" édité par ROY L.WHISTLER, pages 491 à 506 de la seconde édition, ou encore dans l'ouvrage "Starch Production Technology", RADLEY.J.A. (1976) p 175 à 187 et page 178 notamment.

A titre indicatif on trouvera dans le tableau suivant une analyse chimique comparée des amidons de blé A et B tels que l'on peut les trouver dans le commerce.

En tout état de cause, l'amidon de blé B, au sens généralement admis par la profession et qui est celui que lui donne la Demanderesse contient plus de 90 % d'amidon et moins de 5 % de protéines.

Il ne saurait donc s'agir du résidu à faible matière sèche et contenant moins de 70 % d'amidon qui a été déshydraté puis hydrolysé pour servir de substrat à la fermentation citrique décrite dans PREHRAMBENO-TEHNOLOSKA BIOTEHNOLOSKA REVIJA, 1994, 32 (1) 17-20. Un tel substrat ne fournit d'ailleurs que de piètres rendements d'acide citrique et est en réalité le résidu final de l'amidonnerie de blé, comprenant outre l'amidon, l'essentiel des matières solubles de la farine de blé (pentosanes et sucres).

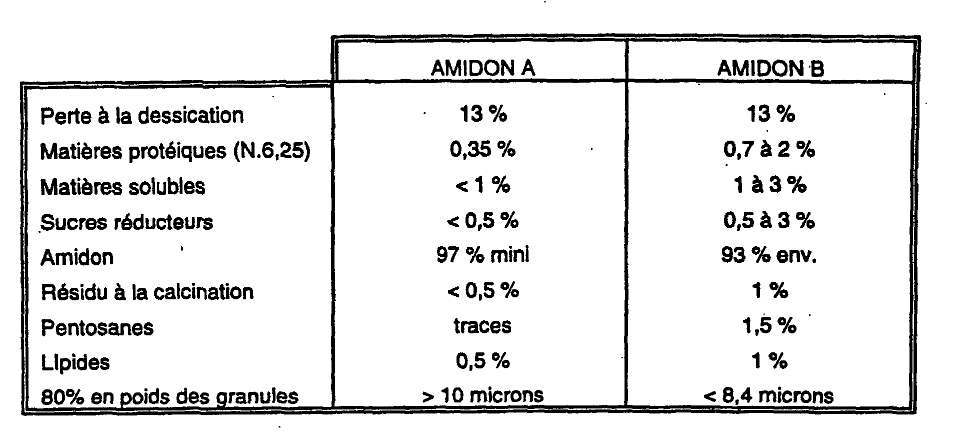

Il s'ensuit que le procédé de fabrication de l'acide citrique conforme à l'invention est caractérisé par le fait qu'au moins une partie de la matière première hydrocarbonée mise en oeuvre pour la fermentation est constituée par un hydrolysat d'amidon B de blé. De préférence, cet hydrolysat représente au moins 25 % de la matière première hydrocarbonée mise en oeuvre, de façon plus préférée, il en représente au moins 50 % et de façon encore plus préférée au moins 75 %.

Par hydrolysat d'amidon, on entend ici les hydrolysats dont la teneur en glucose vrai s'élève à 85 % et davantage de la fraction glucidique de ces hydrolysats. De tels hydrolysats ne peuvent être obtenus qu'avec des amidons de blé B titrant au moins 90 % de richesse en amidon. De préférence, cette teneur en glucose vrai est supérieure à 90 % et de façon encore plus préférée, elle est supérieure à 92 %. Ils peuvent être obtenus par l'hydrolyse acide de l'amidon mais sont de préférence obtenus par l'action d'enzymes amylolytiques telles que l'alpha amylase, l'amyloglucosidase, l'isoamylase ou la pullulanase.

De tels hydrolysats peuvent être mis en oeuvre dans le procédé de l'invention, en employant les techniques de neutralisation des métaux lourds à l'hexacyanoferrate ou au méthanol par exemple, mais selon un mode de réalisation avantageux du susdit procédé, on préfère utiliser un hydrolysat d'amidon uniquement obtenu à base d'amidon B de blé et dont on a enlevé la plus grande partie des métaux lourds par déminéralisation sur une résine cationique forte permutée sous sa forme hydrogène ou éventuellement alcaline ou alcalino-terreuse.

En tant que produits industriels nouveaux, l'invention vise les hydrolysats d'amidon B de blé caractérisés par le fait qu'ils contiennent moins de 500 ppb (partie par billion) de fer et moins de 20 ppb de manganèse, ces teneurs étant exprimées par rapport à la matière sèche des hydrolysats.

De tels hydrolysats d'amidon B selon l'invention peuvent être commercialisés à l'état de sirops déminéralisés sur résine cationique puis concentrés, à la condition d'en enlever la plus grande partie des anions ou de corriger l'acidité apportée par les résines cationiques en la neutralisant au moins partiellement à l'aide d'une base non toxique telle que la soude, la potasse ou l'ammoniaque.

Cette dernière base possède en plus l'avantage d'apporter une partie de l'azote nécessaire à la fermentation.

Ce problème de neutralisation de l'acidité ne se pose pas si les résines cationiques sont utilisées sous cette forme ammoniacale. De telles précautions de neutralisation sont nécessaires parce qu'il est connu qu'en milieu chaud, acide et concentré, le glucose peut se polymériser pour former des oligosaccharides difficilement fermentescibles.

Pour obtenir les hydrolysats d'amidon de blé à mettre en oeuvre dans le procédé de l'invention on peut s'y prendre comme suit ou de manière équivalente.

La liquéfaction d'un lait d'amidon renfermant au moins 25 % d'amidon B de blé est d'abord obtenue par l'action d'une alpha-amylase thermorésistante telle que l'enzyme TERMAMYL commercialisée par la société NOVO. On opère généralement cette liquéfaction à une température comprise entre 65°C et 110°C, à un pH compris entre 5,5 et 6,5, à une matière sèche comprise entre 5 % et 45 %, durant un temps de 1 à 3 heures, avec une dose d'enzyme représentant de 24 unités à 240 unités KNU (Kilo Novo Units) par kilogramme d'amidon de blé, et ce jusqu'à l'obtention d'un DE (Dextrose Equivalent) compris entre 5 et 20 .

La saccharification de cet amidon de blé liquéfié est ensuite obtenue par l'action d'une amyloglucosidase fongique de préférence, telle que l'enzyme DEXTROZYME commercialisée aussi par la société NOVO.

On peut également ajouter d'autres enzymes telles que la pullulanase, l'isoamylase ou des enzymes d'hydrolyse des hémicelluloses. On opère généralement cette saccharification à une température comprise entre 55°C et 63°C, à un pH compris entre 3,5 et 5,5, à une matière sèche comprise entre 5 % et 45 % , durant un temps de 20 à 90 heures, avec une dose d'enzyme représentant de 60 unités à 500 unités G.A.U. (Glucose Amyloglucosidase Unit) par kilogramme d'amidon, jusqu'à l'obtention d'un Dx (pourcentage de glucose vrai) d'au moins 85%.

Au terme de la saccharification, on préfère filtrer les hydrolysats et l'on peut les décolorer et les concentrer comme il est de coutume de pratiquer dans l'industrie amidonnière.

L'hydrolysat d'amidon de blé ainsi obtenu se révèle un substrat particulièrement bien adapté à l'obtention d'acide citrique par voie fermentaire à l'aide d'Aspergillus niger et ce, surtout si cet hydrolysat est réalisé à base d'au moins 25 % d'amidon B de blé.

Il peut être utilisé en l'état, en neutralisant les métaux lourds qu'il contient par l'hexacyanoferrate, l'alcool ou l'addition de cuivre, mais on préfère utiliser un hydrolysat d'amidon B de blé auquel on fait subir un traitement sur résine cationique forte afin d'abaisser à moins de 500 ppb sa teneur en fer et à moins de 20 ppb sa teneur en manganèse, de façon à obtenir le produit de l'invention. Il apparaît que cette manière de faire est celle qui permet de tirer le meilleur parti des substances stimulatrices de la fermentation citrique contenues dans les impuretés des amidons B de blé.

A cette fin, les résines utilisées sont généralement de type à squelette polystyrénique, réticulé au divinylbenzène et fonctionnalisé par sulfonation. De telles résines sont des articles courants du commerce. Il peut par exemple s'agir de la résine C 150 commercialisée par PUROLITE, ou encore de la résine C 200 commercialisée par ROHM and HAAS, ou encore de la résine CM 12 commercialisée par DOW CHEMICALS.

Ces résines sont employées dans les conditions de déminéralisation ou de permutation classiques, préconisées par leurs fabricants.

L'utilisation de l'hydrolysat d'amidon B de blé selon l'invention, à titre de substrat hydrocarboné destiné à la fabrication d'acide citrique, est effectuée conformément aux connaissances générales de l'homme de l'art, telles qu'elles sont rassemblées par exemple dans l'ouvrage "FOOD ACID MANUFACTURE, Recent Developments" de A.A. LAWRENCE, édité par NOYES DATA Corp. édition de 1974 pages 2 à 74 ou encore dans l'ouvrage intitulé "BIOTECHNOLOGY, A Comprehensive Treatise in 8 Volumes", édité par H.J. REHM et G. REED, Volume 3, chapitre 3d, ou encore dans les brochures de la société VOGELBUSCH Ges. m.b.H, AUTRICHE : "INFLUENCE OF NUTRIENT CONCENTRATION IN THE FERMENTATION MEDIA ON PRODUCTION OF CITRIC ACID BY INDUSTRIALLY USED STRAINS OF ASPERGILLUS NIGER".

D'une manière générale, les fermentations sont conduites en cuves aérées et profondes à des concentrations en glucose comprises entre 120 et 200 grammes par litre. Même dans le cas de substrats déminéralisés par échange d'ions, on préfère employer un peu de cuivre et de zinc qui peuvent inhiber le fer ou le manganèse qui pourraient être extraits des parties métalliques des fermenteurs durant la fermentation ou qui s'échappent à l'état de traces des résines. Les seuls nutriments ajoutés sont généralement de l'azote, préférentiellement sous forme ammonium. En effet dans le cas de la fermentation d'hydrolysats essentiellement réalisés à base d'amidon B de blé, leur teneur en impuretés phosphorées est généralement telle qu'il n'est pas nécessaire d'ajouter de phosphates aux moûts de culture. Les fermentations sont conduites à un pH légèrement inférieur à 2,0 à environ 30°C durant 5 à 8 jours jusqu'à disparition quasi totale des sucres réducteurs. L'ensemencement des fermenteurs est généralement assuré par une suspension de spores ou de spores germées d'Aspergillus niger.

Les exemples qui suivent ont pour objet de mieux illustrer l'invention qui vient d'être décrite. Ils ont surtout pour but de mettre en évidence les différences surprenantes et inattendues qui apparaissent dans les comportements des hydrolysats d'amidon de blé A et B, vis à vis de la fermentation citrique par Aspergillus niger en général. Cette différence de comportement est ici illustrée par une souche d'Aspergillus niger adaptée à la fermentation du glucose.

### EXEMPLES :

Des amidons de blé A et B obtenus à partir de la même variété de blé, selon un procédé apparenté au procédé MARTIN tel que décrit dans ses grandes lignes dans l'ouvrage "STARCH : Chemistry and Technology" édité par R.L. WHISTLER, seconde édition 1984, en pages 495-497, ont été hydrolysés dans les conditions identiques suivantes :
- liquéfaction par alpha-amylase de marque TERMAMYL commercialisée par NOVO à raison de 1 ‰ en poids d'enzyme par kilo d'amidon sec, ce qui correspond à 120 KNU par Kg d'amidon dans les conditions suivantes :
   pH = 6,2
   température = 96°C
   durée = 2 h
   jusqu'à un DE de 15,5. Puis saccharification par l'amyloglucosidase DEXTROZYME à raison de 0,85 0/00 d'enzyme par kilo d'amidon dans les conditions suivantes :
   pH 4,5
   température 60°C
   durée 60 heures.

Les sirops obtenus ont tous deux été filtrés sur terre de diatomées puis déminéralisés sur une résine cationique C 200 commercialisée par la société ROHM and HAAS et régénérée sous sa forme hydrogène.

Les hydrolysats d'amidon de blé ainsi obtenus montraient une composition glucidique extrêmement peu différente puisque s'établissant comme suit :

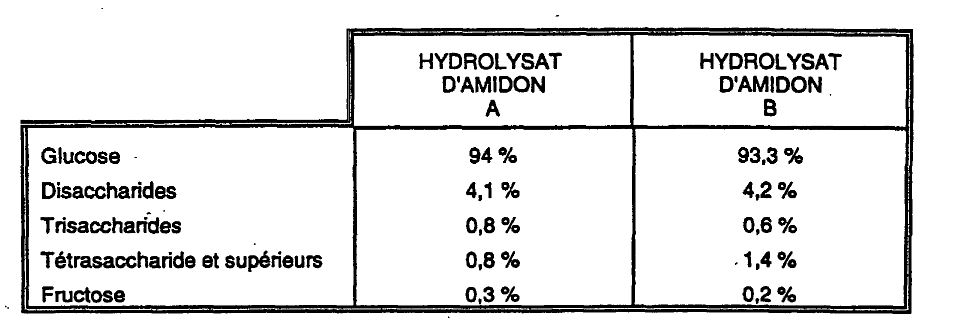

Leur teneur en métaux lourds était très faible puisque s'établissant à moins de 500 ppb de fer et moins de 20 ppb de manganèse.

Les fermentations de ces substrats hydrocarbonés ont été conduites dans des fermenteurs de verre du type "colonne à bulles" équipés de sondes de pH, d'une régulation du pH, d'un contrôle de mousse et d'un système destiné à compenser les pertes dues à l'évaporation.

Ces fermenteurs ont un volume utile de 8,5 litres pour une volume total de 17 litres. Le contrôle de la température est assuré par un bain-marie.

Les hydrolysats d'amidon de blé déminéralisés sont auparavant stérilisés en bouteilles de verre par chauffage durant 30 minutes à la vapeur vive.

Les fermenteurs sont aussi stérilisés à la vapeur vive durant environ 90 minutes puis ils sont refroidis par insuflation d'air stérile.

L'eau de dilution est introduite dans les fermenteurs par l'intermédiaire d'une membrane filtrante stérile de 0,2 micromètre. Cette eau a, au préalable, été déminéralisée.

On ajoute alors les hydrolysats stérilisés et une solution stérile de sels nutritifs de manière à ce que les moûts de culture contiennent (par litre de moût) :
25 ppm de Mg⁺⁺
60 ppm de Ca⁺⁺
100 ppm de K⁺
150 ppb de Fe⁺⁺
500 ppb de Cu⁺⁺
400 ppb de Zn⁺⁺

La concentration en phosphates, des hydrolysats d'amidon B de blé, déminéralisés sur résine cationique, est suffisante pour assurer une croissante correcte du mycélium.

Le pH des moûts de culture est alors porté à 3,5 par l'addition d'une solution d'ammoniaque à 25 % puis est ensuite régulé à pH 1,8 par addition de cette même solution d'ammoniaque tout au cours de la fermentation.

L'ensemencement se fait à l'aide d'une suspension de spores d'une souche dérivée d'Aspergillus niger ATCC 11414, accoutumée à fermenter le glucose.

Le taux d'inoculation est de 0,7 mg de spores par litre de moût à fermenter.

La température des fermenteurs est maintenue à 30°C durant la phase de production de l'acide citrique. Celle-ci peut être plus élevée au moment de 1'inoculation" de façon à obtenir une germination plus rapide des spores. Les fermenteurs ont été aérés au débit de 70 l/mn durant les seize premières heures puis au débit de 90 l/mn durant tout le cours de la fermentation.

Des échantillons ont été régulièrement prélevés tout au cours des fermentations et on a mis fin à celles-ci lorsque le taux de glucose résiduel était inférieur à 2 g/l. Qu'ils proviennent d'un amidon A de blé (exemples comparatifs) ou d'un amidon B, (exemples selon l'invention), les substrats ont été testés à diverses concentrations initiales et les résultats les plus représentatifs ont été rassemblés dans le tableau suivant.

On a fait figurer dans ce tableau les concentrations initiales en, glucose des moûts de culture, la durée totale de la fermentation, la concentration finale en acide citrique après correction des pertes dues à l'évaporation, la teneur finale en biomasse des moûts, la productivité obtenue en grammes d'acide citrique monohydrate par litre de moût et par heure et le rendement pondéral en acide citrique monohydrate par rapport au glucose mis en oeuvre.

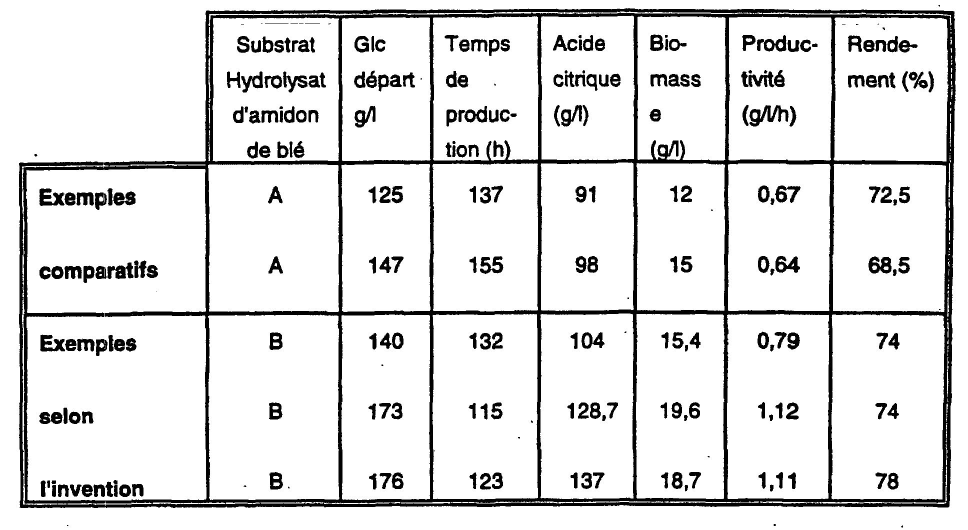

On a constaté qu'en augmentant la teneur en glucose à des valeurs supérieures à 120 g/l, on obtenait une baisse corrélative des rendement et productivité lorsque le substrat est constitué par un hydrolysat d'amidon A de blé.

Au contraire on constate que le procédé et l'hydrolysat d'amidon B de blé selon l'invention, permettent d'obtenir de façon surprenante et sans qu'on sache pourquoi, des productivités et des rendements accrus d'acide citrique, et ce phénomène est d'autant plus marqué que l'on augmente jusqu'à des valeurs proches de 180 grammes par litre la concentration en glucose des moûts de culture.

Des hydrolysats mixtes d'amidon de blé A et B fournissent des résultats intermédiaires qui confirment que les amidons de blé B ou les hydrolysats d'amidon de blé B contiennent des substances stimulatrices de la fermentation citrique.

De même, des hydrolysats d'amidon B de blé employés en mélange avec des hydrolysats d'amidon de maïs augmentent sensiblement les productivités et concentrations, et à un degré moindre, les rendements d'acide citrique qu'il est possible d'obtenir à partir d'hydrolysats provenant d'amidon de maïs, beaucoup plus pur que l'amidon B de blé.

Ces hydrolysats d'amidon B de blé trouvent ainsi une utilisation particulièrement avantageuse à titre d'agent augmentant les productivité, concentration et rendement d'acide citrique en tant que tout ou partie du substrat hydrocarboné à fermenter.

## Revendications

1. Procédé de fabrication de l'acide citrique par voie fermentaire, **caractérisé par le fait qu'**au moins une partie de la matière première hydrocarbonée est constituée par un hydrolysat d'amidon B de blé contenant au moins 85% de glucose.

2. Procédé de fabrication de l'acide citrique selon la revendication 1, **caractérisé par le fait que** cet hydrolysat représente au moins 25%, de façon préférentielle au moins 50% et de façon encore plus préférentielle au moins 75% de la matière première hydrocarbonée mise en oeuvre.

3. Produit industriel constitué par un hydrolysat d'amidon B de blé, contenant au moins 85% de glucose, ayant subi un traitement sur une résine échangeuse de cations.

4. Produit industriel selon la revendication 3 **caractérisée par le fait qu'**il contient moins de 500 ppb de fer et moins de 20 ppb de manganèse.

5. Produit industriel nouveau selon les revendications 3 et 4 **caractérisé par le fait qu'**il contient au moins 90%, et de préférence au moins 92% de glucose.

6. Application d'un hydrolysat d'amidon B de blé contenant au moins 85% de glucose à la fabrication d'acide citrique par fermentation, à titre d'agent augmentant les productivité, concentration et rendement en acide citrique.

## Patentansprüche

1. Verfahren zur Herstellung von Zitronensäure durch Fermentation, **dadurch gekennzeichnet, daß** wenigstens ein Teil des ursprünglich eingesetzten Kohlenwasserstoffs aus einem B-Getreidestärke-Hydrolysat besteht, welches wenigstens 85 % Glukose enthält.

2. Verfahren zur Herstellung von Zitronensäure nach Anspruch 1, **dadurch gekennzeichnet, daß** das Hydrolysat wenigstens 25 %, vorzugsweise wenigstens 50 % und insbesondere bevorzugt wenigstens 75 % des ursprünglich eingesetzten Kohlenwasserstoffs darstellt.

3. Industrielles Produkt, bestehend aus einem B-Getreidestärke-Hydrolysat, welches wenigstens 85 % Glukose enthält und einer Behandlung auf einem Kationenaustauscherharz unterworfen wurde.

4. Industrielles Produkt nach Anspruch 3, **dadurch gekennzeichnet, daß** es weniger als 500 ppb Eisen und weniger als 20 ppb Mangan enthält.

5. Industrielles Produkt nach Anspruch 3 und 4, **dadurch gekennzeichnet, daß** es wenigstens 90 %, vorzugsweise wenigstens 92 % Glukose enthält.

6. Verwendung eines B-Getreidestärke-Hydrolysats, das wenigstens 85 % Glukose enthält, zur Herstellung von Zitronensäure durch Fermentation, als Mittel zur Steigerung der Ausbeute, Konzentration und des Wirkungsgrades der Zitronenesäure.

## Claims

1. Process for the manufacture of citric acid by fermentation, **characterized in that** at least part of the carbohydrate raw material consists of a wheat starch B hydrolysate containing at least 85% of glucose.

2. Process for the manufacture of citric acid according to Claim 1, **characterized in that** this hydrolysate represents at least 25%, preferably at least 50% and still more preferably at least 75% of the carbohydrate raw material employed.

3. Industrial product consisting of a wheat starch B hydrolysate containing at least 85% of glucose, which has undergone a treatment on a cation exchange resin.

4. Industrial product according to Claim 3, **characterized in that** it contains less than 500 ppb of iron and less than 20 ppb of manganese.

5. New industrial product according to Claims 3 and 4, **characterized in that** it contains at least 90% and preferably at least 92% of glucose.

6. Application of a wheat starch B hydrolysate containing at least 85% of glucose for the manufacture of citric acid by fermentation, as an agent for increasing the productivity, concentration and yield of citric acid.
